# EUROPEAN PATENT APPLICATION

(11) **EP 4 435 150 A1**
(43) Date of publication of application: **25.09.2024**
(21) Application number: 23163291.0
(22) Date of filing: 21.03.2023
(51) Int. Cl.: C25B 3/07, C25B 3/26, C25B 9/21, C25B 15/08, C12P 7/00, C12M 1/00

(54) **CO2 ELECTROLYSIS IN A THREE-COMPARTMENT ELECTROCHEMICAL CELL COMPRISING A FERMENTATION MEDIUM**

(71) Applicant: Nederlandse Organisatie voor toegepast-natuurwetenschappelijk Onderzoek TNO, 2595 DA 's-Gravenhage (NL)
(72) Inventor: PEREZ GALLENT, Elena, 2595 DA' s-Granvenhage (NL); GOETHEER, Earl Lawrence Vincent, 2595 DA' s-Granvenhage (NL); VAN DER WILLIGE Nolan, 2595 DA' s-Granvenhage (NL)
(74) Representative: V.O.

(57) **Abstract**

The invention is directed to an integrated CO₂ reduction and fermentation method and an apparatus therefor, said method comprising reducing carbon dioxide in a three-compartment electrochemical cell to produce obtain a carbon dioxide reduction product; leading a lean fermentation medium that is lean in the carbon dioxide reduction product into the extraction compartment of the electrochemical cell and leading a rich fermentation medium that is rich in the carbon dioxide reduction product out of the extraction compartment; and leading the rich fermentation medium into a fermentation unit.

## Description

The invention is in the field of utilizing carbon dioxide (CO₂) based feedstock in fermentation processes. In particular, the invention is directed to an integrated method wherein CO₂ is electrically reduced into a carbon dioxide reduction product and this product is subsequently used in a fermentation process.

CO₂-based feedstock such as formic acid can be used as an energy source for micro-organisms such as bacteria or fungi in fermentation processing to convert typically biological materials to a variety of useful products. Some well-known examples thereof include commodity chemicals such as acetic acid, citric acid and ethanol.

Van Winden et al. (Biotechnol Bioeng. 2022; 119:2142-2151) describe a fed-batch process of feeding formic acid to a fermentation unit containing the yeast Y. lipolytica.

Formic acid may be formed by capturing and electrochemically reducing CO₂, is for instance described in WO2022101182A1. The resulting formic acid can be fed to a fermentation process.

The electrochemical reduction of CO₂ can be carried out in electrochemical cells that use electrical energy to carry out electrochemical reactions. These cells typically comprise at least an anode, a cathode, an electrolyte (e.g. comprising a solvent and an electrolyte solute) and one or more membranes separating the anode and the cathode. An oxidation reaction is performed at the anode and a reduction reaction at the cathode. The products formed at the anode and cathode may be combined to form the final desired product. Particular examples of electrochemical cells include three-compartment cells, having a cathode compartment, an anode compartment and an extraction compartment, which are separated by ion exchange membrane. For instance, the extraction compartment can be separated from the anode by an anion exchange membrane, and the cathodic compartment can be separated from the extraction compartment by a cationic exchange membrane. And vise versa, the extraction compartment may be separated from the anode compartment by a cation exchange membrane and separated from the cathode compartment by an anion exchange membrane, respectively. Examples of such cells are described in *i.a.* WO2019/172750 and US2017/0037522.

A drawback of electrochemically reducing CO₂ to produce carbon dioxide reduction products such as formic acid and leading these products to a fermentation unit is that the reduction product solutions produced by electrochemical reduction do not have a sufficiently high concentration to be directly applied into a fermentation process. Feeding the fermentation process with the diluted product solutions leads to undesired dilution of the fermentation broth. Therefore, an unfavorable energy consuming concentration step is needed after the electrochemical reduction. More specifically, the known methods typically produce the carbon dioxide reduction product in water, resulting in an aqueous solution of the reduced product and a required concentration of at least 65%.

It is an object of the present inventors to provide a method that overcomes at least part of the above-mentioned drawbacks. The present inventors realized that this can be achieved by electrochemically reducing CO₂ and directly extract the reduction product thereof in a fermentation medium.
Figure 1 is a schematic illustration of a three-compartment electrochemical cell suitable for the present invention.
Figure 2A-2B schematically illustrates the formation of formic acid in a three-compartment electrochemical cell.
Figure 3 schematically illustrates an integrated system according to the present invention comprising a fermentation unit and a three-compartment electrochemical cell.
Figure 4A schematically illustrates a preferred system comprising two buffer vessels (30, 50).
Figure 4B schematically illustrates a preferred system comprising a separation unit (40).
Figure 5A schematically illustrates a preferred system comprising a separation unit (30), buffer vessels (30, 50) and a third conduit (103).
Figure 5B schematically illustrates a preferred system comprising a separation unit (40), buffer vessels (30, 50) and a fourth conduit (104).
Figures 6-9 illustrates the results of the examples as detailed herein.

Thus, in a first aspect, the present invention is directed to an integrated CO₂ reduction and formation method wherein electrochemical CO₂ reduction and fermentation are directly integrated. This method accordingly comprises:
- reducing carbon dioxide in a cathode compartment of a three-compartment electrochemical cell to produce an anionic carbon dioxide reduction product;
- producing a cationic oxidation product in an anode compartment of the electrochemical cell;
- transporting said carbon dioxide reduction product anions through an anion exchange membrane (AEM) to an extraction compartment of the electrochemical cell and transporting said cationic oxidation product through a cation exchange membrane (CEM) into the extraction compartment of said electrochemical cell to obtain said carbon dioxide reduction product;
- leading a lean fermentation medium into the extraction compartment that is lean in the carbon dioxide reduction product and leading a rich fermentation medium that is rich in the carbon dioxide reduction product out of the extraction compartment;
- leading the rich fermentation medium into a fermentation unit.

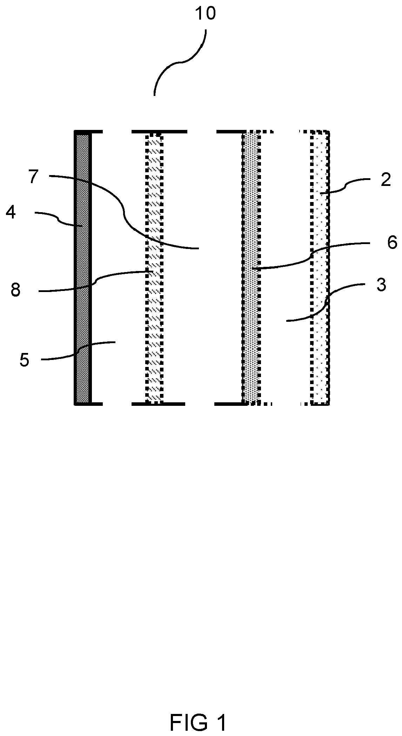

A suitable three-compartment electrochemical cell that can be used in this method is illustrated in Figure 1. The electrochemical cell (10) has a cathode (2) in a cathode compartment (3) and an anode (4) in an anode compartment (5). The electrochemical cell further has an extraction compartment (7), which is separated from the anode compartment by a cation exchange membrane (CEM) (8). The extraction compartment is separated from the cathode compartment by an anion exchange membrane (AEM) (6). Suitable three-compartment electrochemical cells, cathodes, anodes, AEM, CEM, catholytes and anolytes are described in WO2019/172750 and WO2021/256931, which are both incorporated herein in its entirety.

The extraction compartment is thus provided between the CEM and AEM. The extraction compartment comprises a fermentation medium.

Herein, the terms "product" and "carbon dioxide reduction product" are used interchangeably. Further, the terms "three-compartment electrochemical cell" and "electrochemical cell" are herein used interchangeably. The term "anionic carbon dioxide reduction product" is herein interchangeably used with the term "anionic product".

Directly providing the fermentation medium in the electrochemical cell allows for a variety of advantages, besides eliminating the need for a concentration and/or separation step of the product. For instance, the method can be continuous, thereby improving overall process efficiency.

Fermentation media are known in the art. It may be appreciated that the composition of the fermentation medium can be chosen depending on the micro-organism used and the desired fermentation process. Generally, the fermentation medium comprises at least carbohydrates, salts and/or metals. For instance, the fermentation medium may comprise glucose, nitrate salts, and/or one or more trace metals such as iron and zinc.

An example of a suitable fermentation medium is known as Verduyn medium (Verduyn et al. Yeast, 1992;8(7):501-517). This medium has a pH of 5 and comprises dextrose, (NH4)₂SO₄, KH₂PO₄ and MgSO₄, the vitamins D-biotin, Ca-D-pantothenate, nicotonic acid, myo-inositol, thiamine hydrochloride, pyridoxal hydrochloride and p-aminobenzoic acid, and the trace elements EDTA²⁺, Zn²⁺, Mn²⁺, Co²⁺, Cu²⁺, [MoO₄]²⁺, Ca²⁺, Fe²⁺, [BO₃]³⁻, I.

As used herein, the term 'lean fermentation medium' means that this medium contains less of the carbon dioxide reduction product than the 'rich fermentation medium'. The lean fermentation medium is provided in the extraction compartment wherein it is part of the middlelyte, *i.e.* the electrolyte that is present in this compartment. In this compartment, the lean fermentation medium is enriched with the carbon dioxide reduction product. In all further aspects, the rich and lean fermentation media are typically the same. For instance, both may or may not contain the microorganisms that are used in the fermentation process.

The microorganisms may be sensitive towards the electrochemical conditions in the cell and may therefore be removed before the lean fermentation medium enters the cell and reunited with the rich fermentation medium after this exits the cell. That is, not all micro-organisms are likely to be able to withstand the electrochemical stress that is induced by the electrochemical reduction of CO₂, and it may be preferred that the fermentation medium is separated before it is provided in the extraction compartment. This separation may suitably separate substantially all (*e*.*g*. more than 95%) the micro-organisms. This may be achieved by any suitable separation means such as means for settling, flotation, flocculation, filtration, or centrifugation (see for instance Persson et al., Biotechnol. Bioeng. 2001 72(3):269-77). Preferably, the separation means comprises filtration means, more microfiltration or nanofiltration. Additionally, any solid materials may also be separated, reducing possible damage to the electrochemical cell. The method may according comprise separating micro-organisms from a micro-organisms-containing lean fermentation medium to obtain the lean formation medium before leading said lean fermentation medium into the extraction compartment. The micro-organisms that are separated can be reintroduced to the rich fermentation medium because this is led into the formation fermentation unit.

In the fermentation unit, the rich fermentation medium can be fermented by the microorganisms. The invention is not particularly limited to which type of fermentation process, as long as the reduction product can be utilized in the process. An exemplary fermentation process utilizing formic acid is described in Van Winden et al. (Biotechnol Bioeng. 2022; 119:2142-2151).

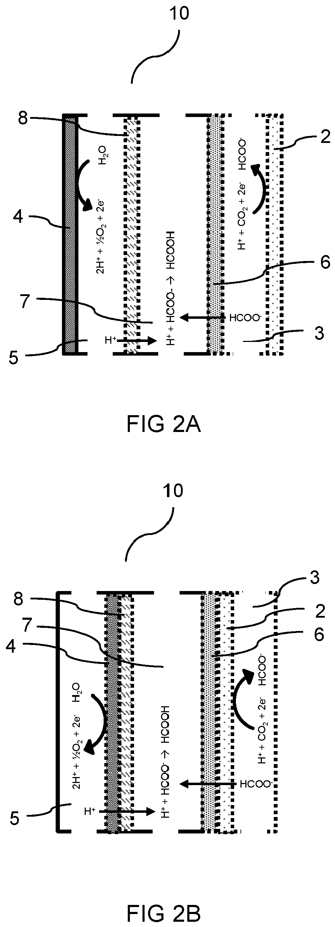

Typical compounds that can be utilized in fermentation process and that can be produced by reducing CO₂ include formic acid, acetic acid, oxalic acid, glycolic acid, tartaric acid, malonic acid, propionic acid, glyoxylic acid, and salts thereof. Accordingly, in a typical embodiment, the carbon dioxide reduction product comprises any one or a combination of this acid and/or salts thereof. Preferably, the product comprises formic acid, and/or a salt thereof. Figure 2A illustrates a preferred embodiment, wherein formic acid is formed from CO₂ and H⁺ in a three-compartment electrochemical cell. The product is formed by the combination of the product anions and cationic oxidation product. Each of which are produced at their respective electrode.

In a typical embodiment, the cation comprises a proton. This may be achieved by the oxidation of for instance H₂, NH₃ and/or H₂O at the anode. The cationic oxidation product may react directly with the anionic reduction product to form the reduction product. Accordingly, in a typical embodiment, the carbon dioxide reduction product anion are allowed to react with the cationic oxidation product in the extraction compartment of said electrochemical cell to obtain the carbon dioxide reduction product. However, other cations, for example inorganic cations such as alkali metals or alkaline earth metals (*e*.*g*. Na⁺, Li⁺, Ca²⁺) may also or alternatively be combined with the product anions to form a salt. This may be particularly so when the process is carried out under alkaline conditions.

Examples of anionic reduction products that can be made are carboxylate ions (R-CO₂-) such as glycolate, oxalate and formate which combine with protons to form glycolic acid, oxalic acid and formic acid respectively. It may appreciated that these carboxylate ions can remain in solution, even when combined with the cationic oxidation product in the extraction compartment.

The cathode may include one or more different catalyst compositions that are either mixed or located in separate regions of the cathode in the cathode compartment.

The cathode structure may comprise one or more selected from the group consisting of platinum, palladium, rhodium, molybdenum, zirconium, niobium, osmium, gold, silver, titanium, copper, iridium, ruthenium, rhenium, mercury, lead, nickel, cobalt, zinc, cadmium, tin, iron, chromium, manganese, gallium, thallium, indium, antimony, and bismuth, oxides and/or alloys thereof, mixed metal oxides, dimensionally stable electrode (DSA^{®}), stainless steel, austenitic steel, ferritic steel, duplex steel, martensitic steel, and carbon-based graphitic electrode.

The preferred cathode structure to electrochemically reduce carbon dioxide to formic acid comprises one or more selected from the group consisting of copper, tin, indium, cadmium, bismuth, mercury, gold, palladium, silver, lead, zinc, and nickel, oxides and/or alloys thereof, and molecular catalysts, such as porphyrins of various metals. The preferred homogenous or metal-complex catalysts to reduce carbon dioxide to formic acid comprises one or more selected from the group consisting of hydrides, halides, or phosphines as ligands.

The preferred cathode structure to electrochemically reduce carbon dioxide to oxalic acid comprises one or more selected from the group consisting of lead, indium, tin, palladium, zinc, titanium, niobium, chromium, iron, thallium, molybdenum, mercury, gallium, graphite, oxides and/or alloys thereof. The preferred homogenous, or metal-complex catalysts to reduce carbon dioxide to oxalic acid comprises one or more selected from the group consisting of low-valent *d*-block and *f-*block metal complexes, copper complexes, nickel complexes.

The preferred cathode structure to electrochemically reduce carbon dioxide to acetic acid comprises one or more selected from the group consisting of copper, iron, and silver, oxides and/or alloys thereof.

The catalyst may be present in the form of nanostructures, such as, nanoparticles and/or nanorods. In addition, the catalyst may be structured as a foam, felt and/or mesh.

To prevent the cation and/or anion exchange membrane from contacting the cathode and/or the anode, a thin material may be applied to the cathode side and/or the anode side of the anion exchange membrane and/or cation exchange membrane, respectively. The thin material may be of plastic origin.

For the electrochemical reduction of carbon dioxide, the cathode material may comprise a coating or a combination of coatings in a single or plurality of layers on the cathode. When a coating or a combination of coatings is present on the cathode, electrochemical reactions may not be inhibited. When the electrode has lost part of its coating, the electrode may be regenerated with subsequent recoating applications. The coating may comprise one or more species selected from the group consisting of alkali metals, alkaline earth metals, lanthanides, actinides, transition metals, post-transition metals, and metalloids, oxides and/or alloys thereof, mixed metal oxides, and ion-conductive polymers. An example of such an ion-conductive polymer is sulfonated tetrafluoroethylene-based fluoropolymer-copolymer. The coating may typically be a very thin layer of several micrometres thick.

The cathode comprises preferably a gas diffusion electrode (GDE) that preferably comprises a catalyst. A GDE is a porous and conductive electrode that in operation preferably provides a conjunction of a solid, liquid and gaseous phase. Typically, GDEs such as carbon-plates, reticulated vitreous carbon (RVC), carbon particles and/or carbon paper (*e*.*g*. toray paper) carbon cloth are used. Additionally, these may be doped with various elements (*e*.*g*. N-doping, O-doping) to enhance the catalytic activity. Other suitable catalyst materials are provided above.

The GDE cathode preferably comprises a current collector such as a metal mesh, for example nickel, platinum, iridium oxide, gold-plated nickel wire mesh or stainless-steel wire mesh, or carbon paper or carbon fleece. The current collector is preferably positioned at the gas stream side (*i.e.* opposite side from the extraction compartment) of the GDE cathode.

The anode structure may also include one or more different catalyst compositions that are either mixed or located in separate regions of the anode structure in the anode compartment. The anode structure and/or catalyst compositions may comprise the material as mentioned above, concerning the cathode, cathode material, coating, and the catalyst compositions. The anode may comprise at least one electrocatalytic coating to be applied to the surface of the anode structure.

The anode is preferably stable against corrosion, mechanically stable and has a uniform current distribution. The anode may comprise lead oxide, for instance PbO₂, which may be supported on a metal such as lead, or on porous graphite such as activated carbon, carbon nanotubes (CNT), reticulated vitreous carbon (RVC) or carbon felt or a titanium support, or a boron doped diamond (BDD). The anode may be a 2D or 3D structure. Particularly high conversion rates may be achieved by using the preferred porous electrodes, fusion electrodes, mesh electrodes, nanostructured electrodes, metal or metal oxide particles supported on porous carbon/graphite electrodes or a combination thereof. The anode may be a gas diffusion electrode (GDE).

Suitable shapes of the electrodes are for example plate, mesh, foam, rod, wire and/or ribbon. Preferably the cathode and/or anode are plate shaped with a relatively small thickness compared to their length and width. They preferably have a plate-like shape that may be flat, curved, rolled or tubular.

The anode and/or cathode of the electrochemical cell may be heated locally. By heating one or both electrodes carbon dioxide may be locally released from the carbon dioxide-rich absorbent (*vide infra*), and directly react on the electrode. Consequently, the reaction rate of carbon dioxide reduction may be accelerated.

The economic viability of the method according to the invention can be optimised by improving the Faradaic conversion efficiency for the desired carbon dioxide reduction product, sufficient high current density (reaction rate), and minimal over potential. In order to calculate the Faradaic efficiency, the number of electrons transferred, total amount of the used carbon dioxide or produced carbon dioxide reduction product, and amount of charge passed through the electrochemical cell are required.

A suitable way of enhancing the efficiency, is by improving catalytic activity, reducing over potential and improving the surface area of the anode and/or cathode. The surface area may be 1-200 m²/g for a catalyst or 1-250 m²/g for a catalyst support. The surface area is defined as the total surface area of a material per unit of mass. The advantage in using a high electrode surface area is that the current flow increases. Typically, catalysts have surface area of 20-100 m²/g for metal catalysts and 5-250 m²/g for activated carbon supports. For most applications, a high surface area is preferred. However, a high surface area at the same time leads to a lower stability of materials. Therefore, an optimum can be reached for each material.

The surface structure of the anode and/or cathode may have a void volume in the range of 1-99 % by total volume. The void volume percentage refers to the percentage of "empty space" that the electrode is not occupying of its total volume space. The void volume has influence on the pressure drop of the electrode for liquid flow through its structure. In general, the higher the void volume, the lower the pressure drop. In particular, the void volume of the anode and/or cathode may be 98 vol.% or less, 5 vol.% or more, 10 vol.% or more, 15 vol.% or more, 20 vol.% or more, 25 vol.% or more, 30 vol.% or more, 35 vol.% or more, or 40 vol.% or more. Preferably, the void volume of the anode and/or cathode is 30-98 vol.%. Low void volume, or low porosity of material, undesirably leads to lower surface area and higher material costs.

In order to improve the mechanical properties of the electrochemical cell, the AEM and/or the CEM may be mechanically supported.

The AEM and/or the CEM may be in direct electrical contact with the cathode and/or anode, respectively, of the electrochemical cell. The respective membrane may be hot-pressed onto the cathode and/or anode of the electrochemical cell or where the cathode and/or anode is deposited on the respective membrane. Alternatively, the respective membrane may be in indirect electrical contact with the cathode and/or anode of the electrochemical cell *via* an electrically conductive substance, such as an electrolyte, or more specifically a catholyte or anolyte. Preferably, the distance between the membranes and cathode and anode is minimal, or the membranes are in direct contact with the respective electrode in order to minimise electrical resistance.

The produced cationic oxidation product and product anions are transported trough a cation exchange membrane and an anion exchange membrane, respectively. The cationic oxidation product may be transported through the CEM and the product anions through the AEM by applying an electrical field to the anode and cathode. The transport of the ions may involve diffusion, migration and surface site hopping.

The CEM that is used in the method according to the present invention is a selective cation-permeable membrane. Accordingly, at least a part of the produced cationic oxidation product is transported through the membrane into the extraction compartment. The CEM may comprise one or more polymers that comprise fixed anionic groups. This typically promotes selective permeability for cationic oxidation product and blocks the passage for anions.

Suitable materials for the CEM are polymers comprising groups such as SO₃-, COO-, PO₃-, HPO₃-, salts thereof and/or acids thereof. The CEM is preferably based on perfluorosulfonic acid, more preferably on perfluorosulfonic acid / polytetrafluoroethylene (PTFE) copolymers in acid form. Particularly suitable polymers comprise perfluorovinyl ether groups that are terminated with sulfonate groups and are incorporated onto a tetrafluoroethylene backbone. Examples thereof are the Nafion^{®} membranes available from DuPont, such as N112, N115 and N117. Other suitable membranes include but are not limited to CM1, CM2, CMB, CMS, CMX and CMXSB available from Eurodia and/or Astom Corporation.

The AEM is a selective anion-permeable membrane that may block the passage of cations. Accordingly, at least part of the produced product anions is transported through the membrane into the extraction compartment. The AEM typically comprises one or more polymers that comprise fixed cationic groups.

Suitable materials for the AEM are polymers that for instance comprise groups such as RH₂N⁺, R₂HN⁺, R₃P⁺, R₂S⁺. The groups may be covalently bound to the polymeric backbone. It is preferred that the AEM is sufficiently base resistant. Particularly suitable polymeric materials comprise a polyolefin backbone with tetraalkyl ammonium groups. Preferred membranes may include the Tokuyama Neosepta, AHA, ACM, ACS, AFX, AM1, AM3, AMX membranes available from Astom Corporation and/or Eurodia. Additionally, the FAA, FAB, FAD, FAS and FTAM membranes available from Fumatech are also suitable. Quaternary ammonium groups on cross-linked fluorinated polymers, such as Morgane^{®} ADP membrane from Solvay or a perfluoro-AEM such as Tosflex^{®} from Tosoh Co may also be suitable. The most preferred membrane is the AHA membrane in view of its chemical stability and selectivity.

The permselectivity of the AEM is preferably 0.9 or more, more preferably 0.95 or more, most preferably 0.98 or more. The permselectivity is a quantity that is used to describe the ability of a membrane to distinguish anions and cations. It may be determined from the percentage calculated from the concentration potential that is developed between solutions of the same electrolyte at different concentrations that are separated by the membrane as described by W. Grot, Fluorinated Ionomers (second edition), 2011. AEM with such selectivity are commercially available, for example the AHA membrane available from Eurodia and Astom. Furthermore, the AEM is for example less than 1 mm thick, or less than 0.5 mm and may for instance be provided with fiber reinforcement. The permselectivity is preferably sufficient to provide negligible permeation of cations through the AEM. Similarly, the permselectivity of the CEM may be sufficient to provide negligible permeation of anions.

Preferably the AEM and/or CEM are plate-shaped with a relatively small thickness compared to their length and width. They preferably have a plate-like shape that may be flat, curved, rolled or tubular.

Reduction of carbon dioxide may also be performed in an electrochemical cell comprising a membrane-electrode assembly (MEA). This may also be referred to as zero-gap type electrochemical cells. These are known to the skilled person. A zero-gap type electrochemical cell is an electrochemical cell, wherein an electrode is adjoined with the ion-selective membrane. An electrode which is in direct contact with the membrane is referred to as zero-gap type electrode. Preferably, the zero-gap type electrode is a GDE. In this arrangement, redox reactions take place at the interface between the zero-gap type electrode and the membrane or in the pores of the electrode. The electrochemical reduction of carbon dioxide takes place at the interface between the cathode and the membrane or in the pores of the cathode. By using one or more zero-gap type electrodes, energy savings can be achieved, because voltage losses are minimised due to the reduced distance between the electrodes.

The membrane and electrode are preferably adjoined face-to-face with the membrane on the side in contact with the extraction compartment. Accordingly, it is preferred that the electrode and membrane both have a plate-like shape. Typically, the membrane and electrode are adjoined at a side surface of each, as opposed to at an edge. Preferably the electrode and the membrane are in contact, preferably in touching contact, with each other over at least 90% by area of a side of each, more preferably over 95% or more.

The electrode and the membrane are preferably stacked on each other to form a multilayer structure of generally parallel layers. One layer comprising or formed by the electrode and a next layer comprising or formed by the membrane. The MEA may be formed by clamping, (hot-)pressing, adhering and/or gluing, preferably by hot-pressing. Alternatively, the membrane may be formed on the electrode by casting or incorporating ion exchange particles into the top layer of the electrode that faces the inside of the electrochemical cell. Alternatively, the electrode may also be formed on the membrane.

The MEA may comprise one or more elements that attach the membrane and the electrode together, such elements may include clamps and/or adhesives. Another way of assuring good contact between the membrane and electrode is by applying a higher pressure in the electrochemical cell that presses the membrane onto the electrode.

The membranes and/or electrodes are typically arranged in a planar arrangement, such as in an essentially parallel plate arrangement. They may also be arranged in a concentric arrangement, such as in a circular configuration or in a spirally wound configuration. The electrode is typically in liquid contact with the corresponding membrane. The CEM is typically positioned between the anode and the AEM and the AEM is typically positioned between the cathode and the CEM. An example, including a preferred conversion to formic acid, is illustrated in Figure 2B. Herein, the CEM (8) is adjoined to the anode (4) and the cathode (2) is adjoined to the AEM (6).

The extraction compartment of the electrochemical cell comprises a liquid, *i.e.* the fermentation medium. The cathode compartment and/or anode compartment may each individually be a gas-compartment. For instance, when the cell comprises one or more GDEs. These gas-compartments are typically adjacent to the electrode on the gas stream side of the electrode (*i*.*e*. the side opposite of the extraction compartment). The gas-compartment may be employed to provide a gas stream (*e*.*g*. a CO₂ -rich gas) to the electrode.

The CO₂ may be introduced in the cathode compartment by providing a CO₂-rich gas stream, for instance originating from flue gases or CO₂ capture plants. It may be preferred that the CO₂ is introduced in the cathode compartment by a carbon dioxide-rich absorbent. This carbon dioxide-rich absorbent may be produced by contacting a carbon dioxide-containing gas stream with an absorbent, thereby absorbing carbon dioxide from the carbon dioxide-containing gas stream. In such cases, CO₂ will be extracted from the CO₂-rich absorbent, leaving a CO₂-lean absorbent. This CO₂-lean absorbent may recycled.

In order for the electrochemical cell to function properly, it is typically required that the cathode, anode and/or extraction compartment comprises a conductive material (*i.a.* an electrolyte) to allow for ions to flow.

For the cathode and/or anode compartment any conventional electrolyte may be used. These can be referred to as a catholyte and anolyte for the cathode compartment and the anode compartment, respectively. The electrolyte in the extraction compartment is herein also referred to as middlelyte. Particularly suitable catholytes and anolytes are provided in WO2019/172750.

The catholyte is preferably liquid or gaseous, and most preferably the catholyte is liquid. Suitable examples of catholytes may comprise potassium bicarbonate solution, potassium hydroxide, monoethanolamine, diethanolamine, diisopropanolamine, N-methyldiethanolamine, diglycolamine, aminomethylpropanol, and/or ammonia, and water.

The catholyte may be substantially non-aqueous. A substantially non-aqueous catholyte is defined herein as a catholyte comprising less than 10 wt.% water, preferably less than 5 wt.% water, further preferably less than 3 wt.% water, such as less than 1 wt.% water. In an embodiment the catholyte is completely free from water.

To increase electrical conductivity and/or regulate the pH value of the catholyte, acids, bases, and/or salts may be added.

The catholyte may comprise an organic and/or inorganic acid. An inorganic acid may be preferred since inorganic acids may be more inert compared to organic acids. Preferably, the inorganic acid may comprise one or more selected from the group consisting of hydrochloric acid, carbonic acid, nitric acid, phosphoric acid, sulphuric acid, boric acid, hydrofluoric acid, hydrobromic acid, perchloric acid, or hydroiodic acid.

When a basic, or alkaline, compound is selected to be added to the catholyte, the basic compound may comprise one or more selected from the group consisting of lithium hydroxide, sodium hydroxide, potassium hydroxide, rubidium hydroxide, caesium hydroxide, magnesium hydroxide, and calcium hydroxide.

Alternatively, or additional to acids and bases, the cathode compartment of the electrochemical cell may further comprise one or more salts. The salt may comprise one or more selected from the group consisting of alkali metal bicarbonates, carbonates, sulphates, phosphates, borates, and/or hydroxides, sodium sulphate, potassium chloride, sodium nitrate, sodium chloride, sodium fluoride, sodium perchlorate, potassium perchlorate, potassium silicate, calcium chloride, guanidinium cation, guanidinium derivatives, hydrogen cation, alkali metal cation, ammonium cation, alkylammonium cation, tetraalkyl ammonium cation, halide anion, alkylamine, borate, carbonate, nitrate, nitrite, phosphate, polyphosphate, perchlorate, silicate, sulphate, or hydroxide. In particular, potassium bicarbonate, sodium bicarbonate, sodium bromide, potassium bromide, and/or salts of the above-mentioned acids and bases are preferred. The at least one salt may be present in the cathode compartment in a concentration in the range of 1-50 % by total weight.

The catholyte may alternatively or additionally comprise one or more selected from the group consisting of (various) dimethyl ethers of polyethylene glycol, *N*-methyl-2-pyrrolidone, methanol, alkylene carbonates such as propylene carbonate, acetone, sulpholane, dimethylsulphoxide, tetrahydrofuran, dimethylformamide, N-methyl-2-pyrrolidone, hexamethylphosphoramide, acetonitrile, dichloromethane, propylene carbonate, pyridine, hexafluoro-2-propanol, and/or ionic liquids comprising 1-butyl-3-methylimidazolium and hydrogen sulfate, trifluoroacetate, dihydrogen phosphate, chloride, nitrate, tetrafluoroborate, triflate and/or hexafluorophosphate.

The cathode compartment of the electrochemical cell may preferably comprise an alkaline, or basic, environment. Herewith, the catholyte in the cathode compartment has a pH value between 7-14.

Depending on the catholyte, formation of various side-products, such as gaseous H₂, might occur. When a substantially non-aqueous catholyte is used, selectivity toward oxalic acid, glyoxylic acid, and/or glycolic acid may be increased. Without wishing to be bound by theory, this may be caused by dimerisation of CO₂⁻-radicals formed by reduction of CO₂.

Another effect of using a substantially non-aqueous catholyte may be that side-reactions leading to H₂ evolution are less favoured, thereby making CO₂ reduction more selective toward other products. These products may include oxalic acid, glyoxylic acid, glycolic acid, tartaric acid, malonic acid, and/or propionic acid.

If unsaturated molecules, such as alkenes or alkynes, are present in a non-aqueous catholyte, CO₂⁻-radicals formed by reduction of CO₂ may react with the unsaturated molecules, resulting in formation of a mixture of mono- and dicarboxylic acids.

The anolyte may be liquid or gaseous. Preferably the anolyte is liquid, more preferably comprising water. The anolyte may include alkali metal hydroxides, such as lithium hydroxide, sodium hydroxide, potassium hydroxide, and ammonium hydroxide, inorganic acids such as sulphuric acid, and hydrochloric acid, organic acids such as methanesulphonic acid, and solutions of alkali halide salts such as sodium chloride, lithium bromide, and sodium iodide. Protic and aprotic organic and inorganic solvents may be used as well as the anolyte. Selection of the anolyte may be dependent by the desired carbon dioxide reduction product. The anolyte may comprise the same above-mentioned material as the catholyte.

The anode compartment of an electrochemical cell may further comprise at least one salt. The salt may preferably be an ionic complex comprising at least one alkali metal or alkaline earth metal and at least one anionic species. The salt may comprise one or more materials as mentioned above with the cathode compartment. The at least one salt may be present in the cathode compartment in a concentration in the range of 1-50 % by total weight.

The extraction compartment comprises the fermentation medium. Depending on the composition of the fermentation media, the fermentation media may be sufficiently conductive to function on its own as middlelyte. However, in some cases it may be preferred to increase the conductivity and reduce the (electrical) resistance. This may be achieved by providing an additional electrolyte component in the extraction compartment. The type of electrolyte component is not particularly limiting. Some preferred types are dissolved salts that are not detrimental to the fermentation process, or which salts can be separated before introducing the fermentation media in the fermentation unit, and then introduced again in the fermentation liquid as inlet for the electrochemical cell, ion-exchange fillers and/or polyelectrolytes. Accordingly, it is preferred that the extraction compartment further comprises a salt, ion-exchange filler and/or polyelectrolyte.

Ion-exchange fillers used in the art and are typically solid, *e.g.* in the form of beads. The ion-exchange fillers may comprise at least one resin, at least one polymer, or combinations thereof. The fillers may be solid, such as beads, or solubilised, for instance dissolved and/or dispersed in the fermentation medium. An advantage of using solubilised fillers is that the membranes may be placed closer together, resulting in improved electrical conductivity in the electrochemical cell.

Preferably, the extraction compartment comprises a conductive solid resin bead. This is typically in the form of a packed bed structure, which remains in the extraction compartment. The fermentation medium can flow through the resin beads. Solid resin beads in a packed bed structure may be particularly preferred as these do not flow out of the electrochemical cell together with the rich fermentation medium (*vide infra*)*.*

In the embodiments wherein a polyelectrolyte is employed, it is preferred that the polyelectrolyte is dissolved in the fermentation medium. This is typically beneficial to minimize the pressure drop in the electrochemical cell, compared to the use of solid resin beads. Suitable polyelectrolytes are described in WO2021/256931.

The polyelectrolyte may comprise a polymer based on one or more monomers comprising an electrolyte group (*e*.*g*. a carboxylate or amine) and which is capable of conducting ions. The polyelectrolytes do not substantially cross the membranes and can accordingly remain present in the extraction compartment. In other words, the CEM and AEM are accordingly essentially impermeable for the polyelectrolytes, meaning that the electrolyte does not substantially flow to the anode and/or cathode compartments. In other words, at least 95 wt% of the polyelectrolyte present in the extraction compartment does not flow to the anode and/or cathode compartment. For the sake of clarity, even if the polyelectrolytes are circulated through the extraction compartment, this is still regarded that the polyelectrolyte (overall) remains in the extraction compartment. Moreover, the polyelectrolytes are surprisingly sufficiently ion-conductive such that the energy consumption does not undesirably increase. Furthermore, the polyelectrolytes are particularly stable in the extraction compartment and typically non-reactive towards the formed product.

Typical criteria on which the polyelectrolyte may be chosen include ionic conductivity, solubility, stability, ease of separation (*i.a.* molecular weight), toxicity and costs. The polyelectrolyte may be a neutral polymer such as a polyamine. The polyelectrolyte is ion-conductive and may accordingly comprise an anion exchange material, a cation exchange material, an amphoteric material and/or a combination thereof. Preferably the polyelectrolyte comprises a cation exchange material. When an anion exchange material and/or a cation exchange material is dissolved they typically comprise fixed charged groups with mobile counter ions.

In a preferred embodiment, the polyelectrolyte comprises a cation exchange material, *e.g.* an acidic polymer. An acidic polyelectrolyte, *e.g.* a polyelectrolyte having a pKa below 7, increases the stabilities of the products anions. Preferably the polyelectrolyte has a pKa below 6, most preferably below 5 which allows for more dissociated protons in the liquid which lowers the pH and is associated with increased stability of the product.

In another preferred embodiment, the polyelectrolyte comprises a combination of cation exchange materials and anion exchange materials. Typically no specific pKa value is preferred for such a combination of materials. Additionally, no specific pKa value may be desired for a preferred embodiment wherein the polyelectrolyte comprises an amphoteric material.

A suitable polyelectrolyte may comprise a polycationic polymer, a polyanionic polymer or a zwitterionic polymer. More specifically, the polyelectrolyte preferably comprises a polyacrylic acid, a polyethyleneimine, a polyacrylamide, a polystyrene sulfonate, a poly(ethylenesulfonic acid), a polyallylamine, a carboxymethyl cellulose, a polygalacturonic acid, an alginic acid, a polypeptide, a polydiallyldimethylammonium, a chitosan, a polyphosphoric acid, a polymaleic acid, a poly(vinyl sulfonic acid), a polypyridinium, a poly(vinylphosphonic acid), a polyvinylamine, a sodium poly(acrylamide-2-methyl-1-propanesulfoate, a salt thereof, a derivative thereof, a copolymer thereof, and/or a combination thereof. Specific examples include poly(methacrylic acid), poly(4-vinyl-*N*-alkylpyridinium chloride), poly(sodium-4-styrenesulfonate), poly(acrylic acid-co-maleic acid), poly(allylamine hydrochloride), poly(diallyldimethyl ammonium chloride), sodium polyacrylate. Commercially available polyelectrolytes that may also be suitable include but are not limited to Duramax^{™}, Tamol^{™}, Romax^{™} and Dowex^{®} from Dow Chemical, Acusol^{™} and Acumer^{™} from Rohm and Haas, Dispex^{®} and Magnafloc^{®} from BASF and Rheosiove^{™} and Terrablend^{™} from Arkema, Preferably, the polyelectrolyte comprises a polyanionic polymer, as these can be acidic at the right pH. Accordingly, the polyelectrolyte is preferably a polyacid, more preferably a polyacid selected from the group consisting of polyacrylic acids, polystyrene sulfonates, carboxymethyl celluloses, polygalacturonic acids, alginic acids, poly(ethylenesulfonic acids), polyphosphoric acids, polymaleic acids, poly(vinyl sulfonic acids), poly(vinylphosphonic acids), sodium poly(acrylamide-2-methyl-1-propanesulfoates), salts thereof, derivatives thereof, copolymers thereof, and combinations thereof.

A sufficient electrical potential between an anode and a cathode in an electrochemical cell is applied for the cathode to reduce carbon dioxide into a product anion. The electrical potential may be a direct current voltage. The energy source may be configured to implement a variable voltage source. The electrical potential between the anode and cathode may be 10 V or less. In particular, the preferred electrical potential is 0 V or more, and 9 V or less, 8 V or less, 7 V or less, 6 V or less, 5 V or less, 4 V or less, 3 V or less, 2 V or less, or 1 V or less. An electrical potential between 0-5 V is more preferred, and 0-3 V is most preferred. Higher electric potentials, such as more than 9 V, result in higher energy consumption and potentially in degradation of reactor components, such as electrodes.

The method may be carried out at a temperature between 5 and 150 °C, such as between 10-90 °C and at an ambient pressure (approximately 1 bar) or elevated pressures. Elevated pressures such as up to 40 to 60 bar of CO₂ pressure may be suitable for the production of formic acid.

By controlling the electrical potential between the anode and the cathode, and/or by selecting a suitable cathode catalyst or suitable catholyte composition in the electrochemical cell, the desired product or products may be obtained.

The method of the invention may be operated in batch, semi-continuously, or continuously. The method is preferably continuous. This may be achieved by continuously supplying lean fermentation medium and leading rich fermentation medium out of the extraction compartment. When the fermentation medium is in the extraction compartment, carbon dioxide reduction products form in this medium and are allowed to exit the electrochemical cell.

The rich fermentation medium can be directly provided in the extraction compartment from a fermentation unit, after it has been reunited with the micro-organisms that were optionally separated upstream. A fermentation unit is herein used to describe a system that comprises all components suitable for fermentation. This includes, but is not limited to, a vessel comprising a fermentation medium comprising micro-organisms. The fermentation medium that is provided into the extraction compartment may accordingly comprise micro-organisms.

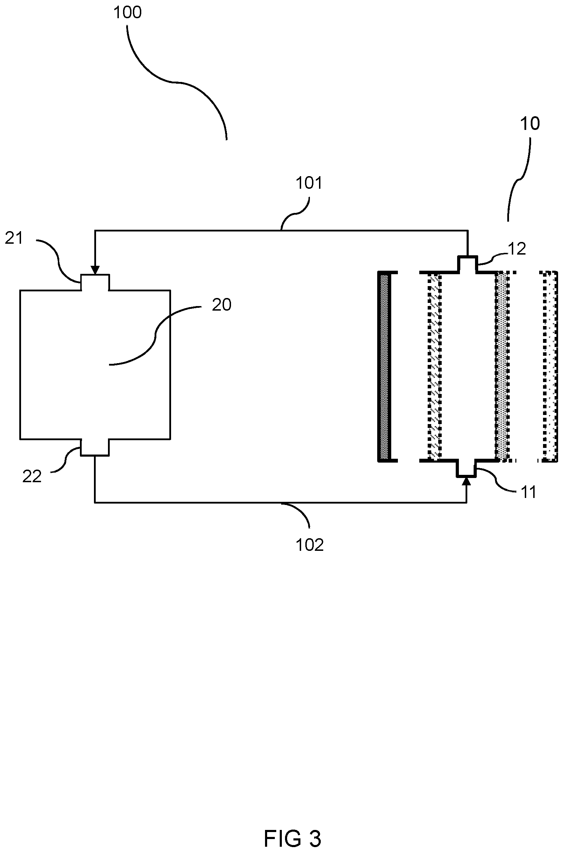

The invention is further directed to an integrated CO₂ reduction and formation system (100) which is suitable to perform the method of reducing carbon dioxide in accordance with the present invention, and of which a particular embodiment is schematically illustrated in Figure 3. The system comprises:
- a three-compartment electrochemical cell (10) for electrochemically reducing carbon dioxide into a carbon dioxide reduction product, wherein the electrochemical cell comprises a lean fermentation medium inlet (11) and a rich fermentation medium outlet (12);
- a fermentation unit (20) comprising a rich fermentation medium inlet (21) and a lean fermentation medium outlet (22);
- a first conduit (101) between the rich fermentation medium outlet (12) and the rich fermentation medium inlet (21);
- a second conduit (102) between the first fermentation medium outlet (22) and the lean fermentation medium inlet (11).

The first and second conduits are typically capable of holding and transporting liquids. For instance, the first conduit allows for the rich fermentation medium to be transported from the extraction compartment to the fermentation unit. Similarly, the second conduit allows for the lean fermentation medium to be transported from the fermentation unit to the extraction compartment.

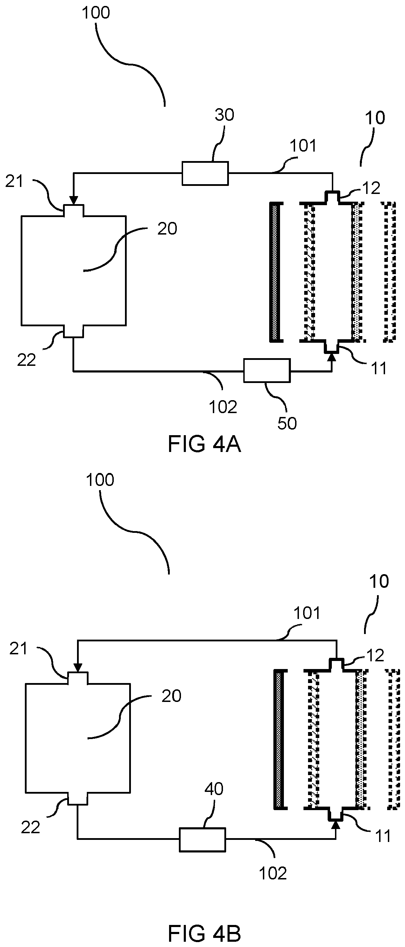

Preferably, the first conduit comprises a first buffer vessel (30) and/or the second conduit comprises a second buffer vessel (50) as illustrated in Figure 4A. These buffer vessels may be used to temporarily store rich and lean fermentation medium before entering and exiting the fermentation unit, respectively. The first buffer vessel may also suitably be used to reintroduce any micro-organisms that have been separated from the fermentation medium before entering the extraction compartment.

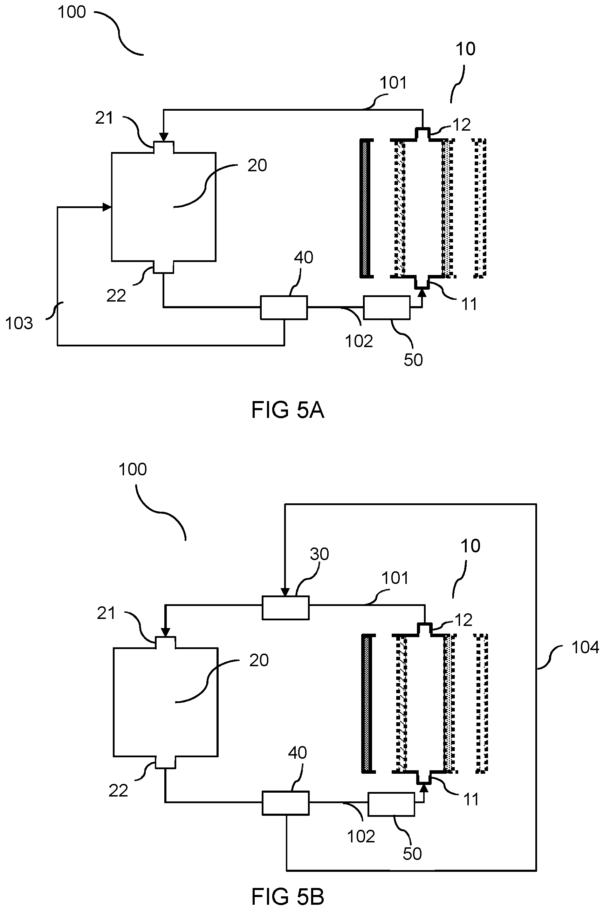

Alternatively, or additionally, the second conduit comprises a separation unit (40) as illustrated in Figure 4B. This separation unit can be used to separate any micro-organisms present in the fermentation medium before entering the extraction compartment. The separation unit can be any conventionally known separation unit, preferably it comprises a filtration unit, more preferably a microfiltration or nanofiltration unit. Any separated micro-organisms may be re-introduced into the fermentation unit, for instance through a third conduit (103), as illustrated in Figure 5A.

In a preferred embodiment, wherein both the buffer vessel and the separation unit are present, the separated micro-organisms may be re-introduced into the fermentation unit, *e*.*g*. through the third conduit (103). Alternatively or additionally be led to the first buffer vessel, thereby bypassing the electrochemical cell, for instance through a fourth conduit (104), as illustrated in Figure 5B.

As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features.

For the purpose of clarity and a concise description, features are described herein as part of the same or separate embodiments, however, it will be appreciated that the scope of the invention may include embodiments having combinations of all or some of the features described.

The present invention can be illustrated with the following nonlimiting examples.

### Example 1 - electrochemical production of formic acid and direct extraction into fermentation medium

### Electrochemical set-up

A 0.2 mol/L KOH solution was made with KOH ACS reagent 85% obtained from Sigma Aldrich. A 0.5 mol/L H₂SO₄ solution was made with H₂SO₄ ACS reagent obtained from Sigma Aldrich. iQ 7003 Milli-Q H₂O was used as solvent. The middlelyte was made by dissolving 50 g of poly(sodium-4-styrenesulfonate), average Mw ~70.000, 30 wt% in H₂O, in 100 g DSM fermentation liquid (*i.e.* Verduyn medium: Verduyn et al. Yeast, 1992;8(7):501-517).

The electrochemical cell used was a 10 cm² Microflow cell containing three-compartments. The catholyte- and extraction-compartment were separated by an anionic exchange membrane (AEM, Fumasep); the anolyte- and extraction compartment were separated by a cationic exchange membrane (CEM, Nafion 117). CO₂ was supplied to the system by a stainless-steel backplate through a 3 mg/cm² Pb-nanoparticle custom carbon cloth GDE (Fuel Cell Etc). An Innovative Instruments, Inc. LF-1 leak-free Ag/AgCl reference electrode was placed inside the catholyte compartment. In all compartments a turbulence promotor was placed.

The catholyte was pumped at 12 L/h; the anolyte was pumped at 3,5-5 L/h; the middelyte was pumped at 18 L/h; and the flowrate of the CO₂ through the humidifier to the GDE was 18 L/h.

Chronoamperometry was run for 24h at -1.8V vs. Ag/AgCl with a 1 second sampling rate.

### Concentration of formic acid over time

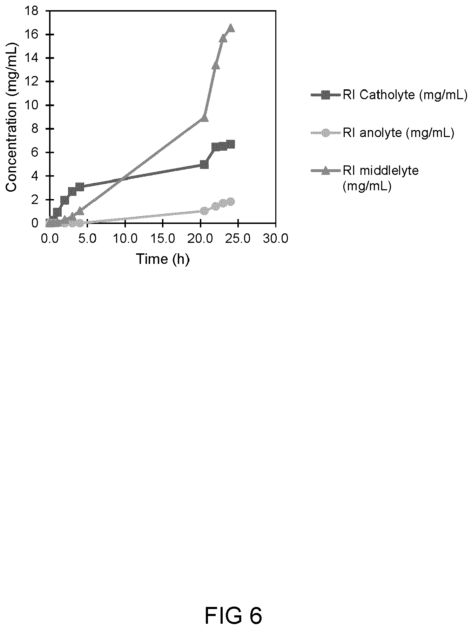

The concentration of formic acid over time, measured with a Waters U-HPLC system containing PDA and RI detector, ran on 5 mM H₂SO₄ solvent, in the catholyte, anolyte and middlelyte are illustrated in Figure 6.

The results show that the concentration of formic acid in the catholyte increases linearly in the first hours, and as the concentration in the catholyte increases the formic acid passes through the AEM to the middlelyte. The catholyte seems to more or less reach a steady-state formic acid concentration after longer runtimes, while the concentration of formic acid in the middlelyte keeps increasing. The middlelyte therefore acts as a formic acid collector.

### pH of the middlelyte

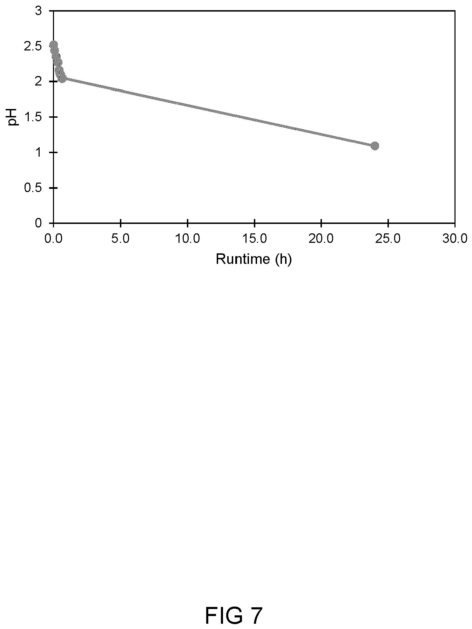

The pH of the middlelyte was tested. Figure 7 illustrates the results. The pH drops over time, as expected due to the acidic nature of formic acid.

### Faradic efficiency of competing reactions

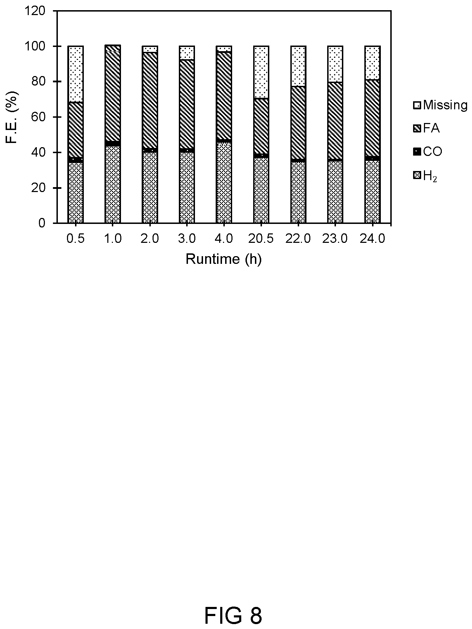

The faradic efficiency (F.E.) of the competing reactions, the hydrogen evolution reaction (HER) and the reduction of CO₂ to CO were calculated to determine the selectivity of the reduction of CO₂ to formic acid. The results are provided in Figure 8. The selectivity towards formic acid at the start of the reaction is about 54%, with around 40 and 2% selectivity towards H₂ and CO respectively. An overall selectivity of around 50% towards FA was found.

### Production rate

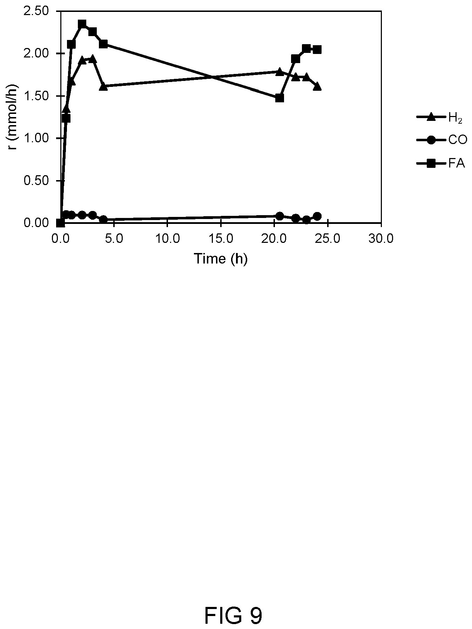

The production rate towards the different products is illustrated in Figure 9. The figure shows that the production of formic acid quickly climbs to about 2 mmol/h and is relatively stable for the complete 24h.

A total middlelyte formic acid volume produced was calculated to be 1.2 mL, which translates to a concentration of 1.4 v%.

## Claims

1. An integrated CO₂ reduction and fermentation method, said method comprising:
- reducing carbon dioxide in a cathode compartment of a three-compartment electrochemical cell to produce an anionic carbon dioxide reduction product;
- producing a cationic oxidation product in an anode compartment of the electrochemical cell;
- transporting said carbon dioxide reduction product anions through an anion exchange membrane (AEM) to an extraction compartment of the electrochemical cell and transporting said cationic oxidation product through a cation exchange membrane (CEM) into the extraction compartment to obtain a carbon dioxide reduction product;
- leading a lean fermentation medium into the extraction compartment that is lean in the carbon dioxide reduction product and leading a rich fermentation medium that is rich in the carbon dioxide reduction product out of the extraction compartment;
- leading the rich fermentation medium into a fermentation unit.

2. Method according to the previous claim, wherein the lean and rich fermentation media comprise carbohydrates, salts and/or metals, preferably dextrose, (NH₄)₂SO₄, KH₂PO₄ and/or MgSO₄.

3. Method according to any of the previous claims, wherein the extraction compartment further comprises a salt, ion-exchange filler and/or polyelectrolyte.

4. Method according to any of the previous claims, wherein the extraction compartment comprises a polyelectrolyte comprising a cation exchange material, an anion exchange material, an amphoteric material and/or a combination thereof.

5. Method according to any of the previous claims, wherein said electrochemical cell is a three-compartment electrochemical cell comprising a membrane-electrode assembly (MEA) and/or catalyst-coated membranes.

6. Method according to any of the previous claims wherein said cathode comprises one or more selected from the group consisting of platinum, palladium, rhodium, molybdenum, zirconium, niobium, osmium, gold, silver, titanium, copper, iridium, ruthenium, rhenium, mercury, lead, nickel, cobalt, zinc, cadmium, tin, iron, chromium, manganese, gallium, thallium, indium, antimony, and bismuth, oxides and/or alloys thereof, mixed metal oxides, dimensionally stable electrode (DSA^{®}), stainless steel, austenitic steel, ferritic steel, duplex steel, martensitic steel, and carbon-based graphitic electrode.

7. Method according to any of the previous claims, wherein said cationic oxidation product comprises a proton.

8. Method according to any of the previous claims, wherein the carbon dioxide reduction product comprises a carboxylic acid, preferably a carboxylic acid selected from the group consisting of formic acid, acetic acid, oxalic acid, glycolic acid, tartaric acid, malonic acid, propionic acid, glyoxylic acid, salts thereof, and combination thereof, more preferably from the group consisting of formic acid and salts thereof.

9. Method according to any the previous claims, further comprising separating micro-organisms from a micro-organisms-containing lean fermentation medium to obtain the lean formation medium before leading said lean formation medium into the extraction compartment, preferably wherein said separating comprising filtering, more preferably nanofiltration.

10. Method according to any of the previous claims, wherein said method is continuous.

11. An integrated CO₂ reduction and formation system (100) for carrying out the method according to any of the previous claims:
- a three-compartment electrochemical cell (10) for electrochemically reducing carbon dioxide into a carbon dioxide reduction product, said electrochemical cell comprising a lean fermentation medium inlet (11) and a rich fermentation medium outlet (12);
- a fermentation unit (20) comprising a rich fermentation medium inlet (21) and a lean fermentation medium outlet (22);
- a first conduit (101) between the rich fermentation medium outlet (12) and the rich fermentation medium inlet (21);
- a second conduit (102) between the first fermentation medium outlet (22) and the lean fermentation medium inlet (11).

12. The system according to the previous claim, wherein said first conduit comprises a first buffer vessel (30) and/or wherein said second conduit a second buffer (50).

13. The system according to any of the previous claims 11-12, wherein said second conduit comprises a separation unit (40) that is suitable to separate microorganisms.
